# EUROPEAN PATENT APPLICATION

(11) **EP 2 487 149 A1**
(43) Date of publication of application: **15.08.2012**
(21) Application number: 10822129.2
(22) Date of filing: 08.10.2010
(51) Int. Cl.: C07C 45/34, C07C 49/17, C07C 49/175, C07C 253/30, C07C 255/17, C07B 61/00

(54) **METHOD FOR PRODUCING KETONE**

(30) Priority: 08.10.2009 JP 2009234556
(71) Applicant: JX Nippon Oil & Energy Corporation, Chiyoda-ku Tokyo 100-8162 (JP); Osaka University, Suita-shi Osaka 565-0871 (JP)
(72) Inventor: KANEDA Kiyotomi, Suita-shi Osaka 565-0871 (JP); SONE Hisashi, Yokohama-shi Kanagawa 231-0815 (JP)
(74) Representative: HOFFMANN EITLE
(86) International application number: PCT/JP2010/067734
(87) International publication number: WO 2011/043459

(57) **Abstract**

A method for manufacturing a ketone, comprising: oxidizing an internal olefin or a cyclic olefin having a functional group containing a hetero atom and one carbon-carbon double bond or more at a position other than terminals of a molecule thereof in an amide-based solvent in the presence of water, a palladium catalyst, and molecular oxygen, without oxidizing the functional group, thereby bonding an oxo group to at least one of the carbon atoms constituting the carbon-carbon double bond, the amide-based solvent being represented by the following formula (1) : 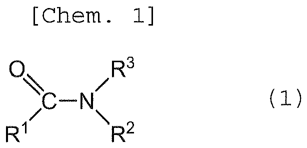 (in the formula (1), R¹ represents an alkyl group having 1 to 4 carbon atoms; R² and R³ each independently represent an alkyl group having 1 to 4 carbon atoms or an aryl group; and when R¹ and R² are alkyl groups, R¹ and R² may be bonded to each other to form a ring structure).

## Description

### [Technical Field]

The present invention relates to a method for manufacturing a ketone by oxidizing an olefin, and more specifically relates to a method of manufacturing a corresponding ketone having a functional group such as hydroxyl group by oxidizing an olefin having the functional group.

### [Background Art]

Carbonyl compounds including ketones such as methyl ethyl ketone (MEK), methyl isobutyl ketone (MIBK), and acetone, and aldehydes typified by acetaldehyde are useful as solvents and chemical raw materials, and hence used in various fields. In addition, ketones having functional groups such as hydroxyl group are expected as solvents having strong cleaning performances, and ketoesters are useful also as pharmaceutical raw materials. Such carbonyl compounds are generally manufactured by two-step reaction methods in which an alcohol produced by hydrating an olefin is dehydrogenated. Meanwhile, as simpler methods, one-step reaction methods have also been known in which an olefin is directly oxidized.

Known methods for directly oxidizing an olefin include the Wacker process in which an olefin is oxidized in the presence of a PdCl₂/CuCl₂ catalyst, a method in which a terminal olefin is oxidized in the presence of a palladium catalyst and molecular oxygen in a polar solvent such as N,N-dimethylacetamide [Angew. Chem. Int. Ed., 2006, 45, 481-485 (NPL 1)], and the like. The Wacker process using a PdCl₂/CuCl₂ catalyst is effective for the oxidation of terminal olefins each having a carbon-carbon double bond (hereinafter abbreviated as a "C=C bond") at a terminal of a molecule thereof. However, the Wacker process has a problem of low reactivity when used for oxidation of internal olefins each having a C=C bond at a position other than its terminals. This process also has a problem that if the number of carbon atoms of the olefin is increased, the reaction rate is markedly lowered. For this reason, in the industrial field, the use of the Wacker process is limited to only the manufacturing of lower carbonyl compounds such as acetaldehyde and acetone, which are obtained by oxidizing lower terminal olefins.

To solve such problems involved in manufacturing a carbonyl compound by direct oxidation of an olefin, various methods have been proposed. For example, Japanese Unexamined Patent Application Publication No. 2002-191979 (PTL 1) discloses a method for manufacturing a ketone, in which an alkene is oxidized with molecular oxygen in the presence of an oxidation catalyst comprising a palladium compound, a heteropolyacid, and a strong acid. Meanwhile, Japanese Unexamined Patent Application Publication No. 2008-207156 (PTL 2) discloses a method for manufacturing a ketone, in which an olefin, molecular oxygen, and water are reacted with each other in the presence of a catalyst comprising a palladium source, a mesoporous silicate, and an isopolyacid or an acidic salt thereof. Moreover, these PTLs disclose those having substituents such as hydroxyl group as the alkenes and the olefins. However, the conventional methods for manufacturing a ketone are still not fully satisfactory from the viewpoint that corresponding ketones are manufactured at high yields and high selectivities from internal olefins and cyclic olefins having functional groups such as hydroxyl group.

### [Citation List]

### [Patent Literature]

[PTL 1] Japanese Unexamined Patent Application Publication No. 2002-191979
[PTL 2] Japanese Unexamined Patent Application Publication No. 2008-207156

### [Non Patent Literature]

[NPL 1] K. Kaneda et al., Angew. Chem. Int. Ed., 2006, 45, 481-485

### [Summary of Invention]

### [Technical Problem]

The present invention has been made in view of the above-described problems of the conventional techniques, and an object of the present invention is to provide a method which makes it possible to oxidize an internal olefin or a cyclic olefin having a functional group such as hydroxyl group so that a corresponding ketone having the functional group can be manufactured at a high yield and a high selectivity.

### [Solution to Problem]

The present inventors have earnestly studied to achieve the above object. As a result, the present inventors have found that when an internal olefin or a cyclic olefin having a functional group containing a hetero atom and one carbon-carbon double bond or more at a position other than terminals of a molecule thereof is oxidized, the use of a specific amide-based solvent in the presence of a palladium catalyst, water, and molecular oxygen makes it possible to bond an oxo group to at least one of the carbon atoms constituting the carbon-carbon double bond, without oxidizing the functional group, so that a ketone which corresponds to the internal olefin or the cyclic olefin, and which has been difficult to manufacture at a high yield and a high selectivity by the conventional methods can be manufactured at a high yield and a high selectivity. This finding has led to the completion of the present invention.

Specifically, a method for manufacturing a ketone of the present invention comprises:
oxidizing an internal olefin or a cyclic olefin having a functional group containing a hetero atom and one carbon-carbon double bond or more at a position other than terminals of a molecule thereof in an amide-based solvent in the presence of water, a palladium catalyst, and molecular oxygen, without oxidizing the functional group, thereby bonding an oxo group to at least one of the carbon atoms constituting the carbon-carbon double bond, the amide-based solvent being represented by the following formula (1): 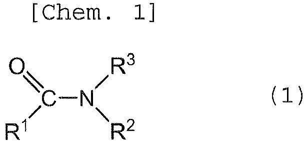 (in the formula (1), R¹ represents an alkyl group having 1 to 4 carbon atoms; R² and R³ each independently represent an alkyl group having 1 to 4 carbon atoms or an aryl group; and when R¹ and R² are alkyl groups, R¹ and R² may be bonded to each other to form a ring structure).

The palladium catalyst is preferably a palladium halide, and a concentration of the palladium catalyst is preferably 0.001 to 1 mol/L. Moreover, the amide-based solvent is preferably N,N-dimethylacetamide.

In the method for manufacturing a ketone of the present invention, the preferred internal olefin or the preferred cyclic olefin is a compound represented by the following formula (2): 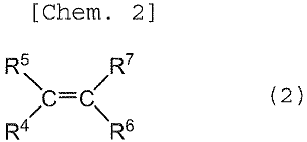 (in the formula (2), at least one of R⁴ to R⁷ is one selected from the group consisting of alkyl groups having the functional group, alkenyl groups having the functional group, and aryl groups having the functional group; the rest of R⁴ to R⁷ are each independently one selected from the group consisting of a hydrogen atom, alkyl groups, alkenyl groups, and aryl groups; at least one of R⁴ and R⁵ is a group other than a hydrogen atom; at least one of R⁶ and R⁷ is a group other than a hydrogen atom; when R⁴ and R⁶ are an alkyl group or an alkenyl group, R⁴ and R⁶ may be bonded to each other to form a ring structure; and when R⁵ and R⁷ are an alkyl group or an alkenyl group, R⁵ and R⁷ may be bonded to each other to form a ring structure). The internal olefin or the cyclic olefin more preferably has the functional group which is bonded to a first to third carbon atom away from the carbon-carbon double bond. In addition, the internal olefin or the cyclic olefin preferably has no carbon-carbon double bond at the terminals of the molecule thereof. Moreover, in the internal olefin or the cyclic olefin, the functional group is preferably at least one functional group selected from the group consisting of hydroxyl group, cyano group, alkoxy groups, acetoxyl groups, and oxo group.

In the method for manufacturing a ketone of the present invention, the internal olefin or the cyclic olefin is preferably oxidized in the absence of any copper catalyst.

Note that, it is not exactly clear why the manufacturing method of the present invention makes it possible to oxidize the internal olefin or the cyclic olefin having a functional group containing a hetero atom so that a corresponding ketone having the functional group can be manufactured at a high yield and a high selectivity. However, the present inventors presume as follows. Specifically, in the method for manufacturing a ketone of the present invention, molecular oxygen is not directly used for oxidation of the internal olefin or the cyclic olefin having a functional group, but is used as a reoxidizing agent. Hence, the functional group such as hydroxyl group is less readily oxidized, and the C=C bond of the olefin is selectively oxidized. Moreover, it is also presumed that the use of molecular oxygen as a reoxidizing agent enables the Wacker reaction of an internal olefin or a cyclic olefin, which has a lower reactivity than those of terminal olefins, to proceed efficiently, so that the yield of the corresponding ketone having the functional group is increased. It is also presumed that, in the method for manufacturing a ketone of the present invention, since no isomerization reaction of the internal olefin occurs during the oxidation of the internal olefin, the selectivity to the corresponding ketone having the functional group is increased.

### [Advantageous Effects of Invention]

According to the present invention, it is possible to oxidize an internal olefin or a cyclic olefin having a functional group such as hydroxyl group so that a corresponding ketone having the functional group can be manufactured at a high yield and a high selectivity.

### [Description of Embodiments]

Hereinafter, the present invention will be described in detail on the basis of preferred embodiments thereof. A method for manufacturing a ketone of the present invention comprises:
oxidizing an internal olefin or a cyclic olefin having a functional group containing a hetero atom and one carbon-carbon double bond or more at a position other than terminals of a molecule thereof in an amide-based solvent in the presence of water, a palladium catalyst, and molecular oxygen, without oxidizing the functional group, thereby bonding an oxo group to at least one of the carbon atoms constituting the carbon-carbon double bond, the amide-based solvent being represented by the following formula (1): 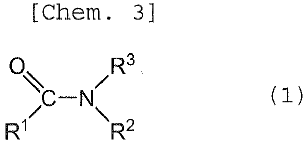 (in the formula (1), R¹ represents an alkyl group having 1 to 4 carbon atoms; R² and R³ each independently represent an alkyl group having 1 to 4 carbon atoms or an aryl group; and when R¹ and R² are alkyl groups, R¹ and R² may be bonded to each other to form a ring structure).

### <Olefin>

The olefin used in the present invention is an internal olefin or a cyclic olefin having a functional group containing a hetero atom and one carbon-carbon double bond or more at a position other than terminals of a molecule thereof (hereinafter referred to as a "functional group-containing internal olefin" and a "functional group-containing cyclic olefin," respectively). Examples of the functional group include hydroxyl group, cyano group, alkoxy groups, phenoxy groups, acetoxyl groups, and oxo group. In an internal olefin or a cyclic olefin having such a functional group, the functional group tends to be less readily oxidized, and an oxo group (=O) tends to be readily bonded to at least one of the carbon atoms in the carbon-carbon double bond. Of the functional groups, hydroxyl group, cyano group, alkoxy groups, acetoxyl groups, and oxo group are preferable, from the viewpoint that the oxo group is likely to be bonded to the carbon atom more distant from the functional group, of the two carbon atoms constituting the carbon-carbon double bond.

In the functional group-containing internal olefin or the functional group-containing cyclic olefin, one kind of functional group alone may be present in one molecule, or two or more kinds of functional groups may be present in one molecule. In addition, when two or more kinds of functional groups are present, the functional groups may be bonded to the same carbon atom, or different carbon atoms.

In addition, in an internal olefin or a cyclic olefin in which the functional group is bonded to a first to third carbon atom (a carbon atom at an allylic position is taken as a first carbon atom) away from the carbon-carbon double bond, the oxo group tends to be bonded more selectively to the carbon atom more distant from the functional group, of the two carbon atoms constituting the carbon-carbon double bond. Particularly in an internal olefin in which the functional group is bonded to a carbon atom at an allylic position, the oxo group tends to be bonded to the carbon atom more distant from the functional group, of the two carbon atoms constituting the carbon-carbon double bond, at a selectivity of 80% or higher.

Moreover, in an internal olefin in which at least one functional group selected from the group consisting of cyano group, alkoxy groups, and acetoxyl groups is bonded to a first to third carbon atom away from the carbon-carbon double bond, the oxo group tends to be bonded to the carbon atom more distant from the functional group, of the two carbon atoms constituting the carbon-carbon double bond, at a selectivity of 90% or higher. Particularly in an internal olefin in which the functional group is bonded to a carbon atom at an allylic position, the oxo group tends to be bonded to the carbon atom more distant from the functional group, of the two carbon atoms constituting the carbon-carbon double bond, at a selectivity of 95% or higher.

The alkyl group in the alkoxy group may be linear, branched, or cyclic, and may have a substituent such as phenyl group, methylphenyl group, or benzyl group. The number of carbon atoms of the alkyl group is preferably 1 to 12, and more preferably 1 to 6. In addition, the phenyl group in the phenoxy group may have a substituent such as an alkyl group, phenyl group, methylphenyl group, or benzyl group.

In the present invention, any olefin having a carbon-carbon double bond at its terminal, or any olefin having no such a bond can be used as the functional group-containing internal olefin or the functional group-containing cyclic olefin according to the present invention, as long as the olefin has the functional group and one carbon-carbon double bond or more at an internal position in a molecule thereof.

The functional group-containing internal olefin or the functional group-containing cyclic olefin is preferably a compound represented by the following formula (2): 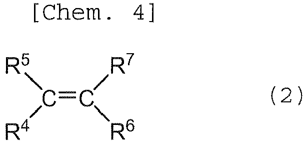 (in the formula (2), at least one of R⁴ to R⁷ is one selected from the group consisting of alkyl groups having the functional group, alkenyl groups having the functional group, and aryl groups having the functional group; the rest of R⁴ to R⁷ are each independently one selected from the group consisting of a hydrogen atom, alkyl groups having no functional group, alkenyl groups having no functional group, and aryl groups having no functional group; at least one of R⁴ and R⁵ is a group other than a hydrogen atom (i.e., any one of the alkyl groups, the alkenyl groups, and the aryl groups having no functional group, and the alkyl groups, the alkenyl groups, and the aryl groups having the functional group); at least one of R⁶ and R⁷ is a group other than a hydrogen atom (i.e., any one of the alkyl groups, the alkenyl groups, and the aryl groups having no functional group, and the alkyl groups, the alkenyl groups, and the aryl groups having the functional group); when R⁴ and R⁶ are an alkyl group or an alkenyl group, R⁴ and R⁶ may be bonded to each other to form a ring structure, and when R⁵ and R⁷ are an alkyl group or an alkenyl group, R⁵ and R⁷ may be bonded to each other to form a ring structure).

The alkyl groups having the functional group and the alkenyl groups having the functional group may be linear, branched, or cyclic. The number of carbon atoms of the alkyl chain (a moiety excluding the functional group) in such an alkyl group having the functional group is preferably 1 to 12. From the viewpoint that a corresponding ketone having a functional group can be manufactured easily by the present invention even when an internal olefin or a cyclic olefin having a long-chain alkyl group is used, the number of carbon atoms of the alkyl chain is more preferably 7 to 12; however, there arises no problem even when the number of carbon atoms of the alkyl chain in the alkyl group having the functional group is 1 to 6. In addition, the position of the C=C bond in the alkenyl group having the functional group is not particularly limited, and may be at a terminal or an internal position. For example, an internal olefin or a cyclic olefin having a C=C bond at a terminal of the alkenyl group having the functional group is a functional group-containing polyene having C=C bonds at terminal and internal positions of a molecule thereof. An internal olefin or a cyclic olefin having a C=C bond at an internal position of the alkenyl group having the functional group is a functional group-containing polyene having two C=C bonds or more at internal positions of a molecule thereof. Examples of the aryl groups having the functional group include groups obtained by substituting the functional group in phenyl group, methylphenyl group, or benzyl group.

In addition, the alkyl groups having no functional group and the alkenyl groups having no functional group may be linear, branched, or cyclic. The number of carbon atoms of such an alkyl group is preferably 1 to 12. From the viewpoint that a corresponding ketone having a functional group can be manufactured easily by the present invention even when an internal olefin or a cyclic olefin having a long-chain alkyl group is used, the number of carbon atoms of such an alkyl group is more preferably 7 to 12; however, there arises no problem even when the number of carbon atoms of the alkyl group having no functional group is 1 to 6. The position of the C=C bond in the alkenyl group is not particularly limited, and may be at a terminal or an internal position of the alkenyl group. For example, an internal olefin or a cyclic olefin having a C=C bond at a terminal of the alkenyl group is a polyene having C=C bonds at terminal and internal positions of a molecule thereof. An internal olefin or a cyclic olefin having a C=C bond at an internal position of the alkenyl group is a polyene having two C=C bonds or more at internal positions of a molecule thereof. Examples of the aryl groups having no functional group include phenyl group, methylphenyl group, and benzyl group.

Moreover, R⁴ and R⁶, and/or, R⁵ and R⁷ may be bonded to each other to form a ring structure. Examples of such a ring structure include cyclic olefins such as cycloalkenes and cycloalkadienes. In such a case, a C=C bond may be present in a moiety other than the ring structure (for example, in R⁵ and/or R⁷ when R⁴ and R⁶ are bonded to each other to form the ring structure).

Specific examples of the functional group-containing internal olefin include hydroxyl group-containing internal olefins such as 2-buten-1-ol [1-hydroxy-2-butene], 2-methyl-2-buten-1-ol [1-hydroxy-2-methyl-2-butene], 3-methyl-2-buten-1-ol [1-hydroxy-3-methyl-2-butene], 2-hexen-1-ol [1-hydroxy-2-hexene], 4-hexen-1-ol [1-hydroxy-4-hexene], 5-methyl-2-hexen-1-ol [1-hydroxy-5-methyl-2-hexene], 2-methyl-4-hexen-1-ol [1-hydroxy-2-methyl-4-hexene], 2-octen-1-ol [1-hydroxy-2-octene], 6-octen-1-o1 [1-hydroxy-6-octene], 3-nonen-1-ol [1-hydroxy-3-nonene], 6-nonen-1-ol [1-hydroxy-6-nonene], 1-(4-hydroxyphenyl)-1-propylene, 1-phenyl-1-propylen-3-ol [3-hydroxy-1-phenyl-1-propylene], 1-(4-hydroxycyclohexyl)-2-butene, and 1-cyclohexyl-2-buten-4-ol [4-hydroxy-1-cyclohexyl-2-butene]; cyano group-containing internal olefins such as 3-pentenenitrile [1-cyano-2-butene], 3-methyl-3-pentenenitrile [1-cyano-2-methyl-2-butene], 4-methyl-3-pentenenitrile [1-cyano-3-methyl-2-butene], 3-heptenenitrile [1-cyano-2-hexene], 5-heptenenitrile [1-cyano-4-hexene], 6-methyl-3-heptenenitrile [1-cyano-5-methyl-2-hexene], 3-methyl-5-heptenenitrile [1-cyano-2-methyl-4-hexene], 3-nonenenitrile [1-cyano-2-octene], 7-nonenenitrile [1-cyano-6-octene], 4-decenenitrile [1-cyano-3-nonene], 7-decenenitrile [1-cyano-6-nonene], 1-(4-cyanophenyl)-1-propylene, 4-phenyl-3-butenenitrile [3-cyano-1-phenyl-1-propylene], 1-(4-cyanocyclohexyl)-2-butene, and 4-cyclohexyl-3-butenenitrile [4-cyano-1-cyclohexyl-2-butene]; alkoxy group-containing internal olefins such as 1-methoxy-2-butene, 1-methoxy-2-methyl-2-butene, 1-methoxy-3-methyl-2-butene, 1-methoxy-2-hexene, 1-methoxy-4-hexene, 1-methoxy-5-methyl-2-hexene, 1-methoxy-2-methyl-4-hexene, 1-methoxy-2-octene, 1-methoxy-6-octene, 1-methoxy-3-nonene, 1-methoxy-6-nonene, 1-(4-methoxyphenyl)-1-propylene, 3-methoxy-1-phenyl-1-propylene, 1-(4-methoxycyclohexyl)-2-butene, 4-methoxy-1-cyclohexyl-2-butene, 1-benzyloxy-2-butene [1-phenylmethyloxy-2-butene], 1-benzyloxy-2-methyl-2-butene [1-phenylmethyloxy-2-methyl-2-butene], 1-benzyloxy-3-methyl-2-butene [1-phenylmethyloxy-3-methyl-2-butene], 1-benzyloxy-2-hexene [1-phenylmethyloxy-2-hexene], 1-benzyloxy-4-hexene [1-phenylmethyloxy-4-hexene], 1-benzyloxy-5-methyl-2-hexene [1-phenylmethyloxy-5-methyl-2-hexene], 1-benzyloxy-2-methyl-4-hexene [1-phenylmethyloxy-2-methyl-4-hexene], 1-benzyloxy-2-octene [1-phenylmethyloxy-2-octene], 1-benzyloxy-6-octene [1-phenylmethyloxy-6-octene], 1-benzyloxy-3-nonene [1-phenylmethyloxy-3-nonene], 1-benzyloxy-6-nonene [1-phenylmethyloxy-6-nonene], 1-(4-benzyloxyphenyl)-1-propylene [1-(4-phenylmethyloxyphenyl)-1-propylene], 3-benzyloxy-1-phenyl-1-propylene [3-phenylmethyloxy-1-phenyl-1-propylene], 1-(4-benzyloxyphenylcyclohexyl)-2-butene [1-(4-phenylmethyloxyphenylcyclohexyl)-2-butene], and 4-benzyloxyphenyl-1-cyclohexyl-2-butene [4-phenylmethyloxyphenyl-1-cyclohexyl-2-butene]; acetoxyl group-containing internal olefins such as 2-butenyl-1-acetate [1-acetoxy-2-butene], 2-methyl-2-butenyl-1-acetate [1-acetoxy-2-methyl-2-butene], 3-methyl-2-butenyl-1-acetate [1-acetoxy-3-methyl-2-butene], 2-hexenyl-1-acetate [1-acetoxy-2-hexene], 4-hexenyl-1-acetate [1-acetoxy-4-hexene], 5-methyl-2-hexenyl-1-acetate [1-acetoxy-5-methyl-2-hexene], 2-methyl-2-hexenyl-1-acetate [1-acetoxy-2-methyl-4-hexene], 2-octenyl-1-acetate [1-acetoxy-2-octene], 6-octenyl-1-acetate [1-acetoxy-6-octene], 3-nonenyl-1-acetate [1-acetoxy-3-nonene], 6-nonenyl-1-acetate [1-acetoxy-6-nonene], 1-(4-acetoxyphenyl)-1-propylene, 1-phenyl-1-propenyl-3-acetate [3-acetoxy-1-phenyl-1-propylene], 1-(4-acetoxycyclohexyl)-2-butene, and 1-cyclohexyl-2-butenyl-4-acetate [4-hydroxy-1-cyclohexyl-2-butene]; alkyl esters (internal olefins in which an oxo group and an alkoxy group are bonded to the same carbon atom) of α,β-unsaturated carboxylic acids (internal olefins in which an oxo group and a hydroxyl group are bonded to the same carbon atom) such as 2-butenoic acid, 2-hexenoic acid, 2-octenoic acid, 2-nonenoic acid, maleic acid, and fumaric acid; and the like. Isomers, such as cis- and trans-isomers, of these functional group-containing internal olefins can be each used equally. Moreover, these functional group-containing internal olefins may be used singly or in combination of two or more kinds.

Meanwhile, examples of the functional group-containing cyclic olefin include those in which the functional group such as hydroxyl group or an oxo group is bonded to a cycloalkene such as cyclopentene, cyclohexene, cyclooctene, or cyclodecene, or a cycloalkadiene typified by cyclooctadiene, and more specifically include oxo group-containing cyclic olefins such as 2-cyclohexen-1-one, hydroxyl group-containing cyclic olefins such as 2-cyclohexen-1-ol, and acetoxyl group-containing cyclic olefins such as 2-cyclohexene-1-acetate. These functional group-containing cyclic olefins may be used singly or in combination of two or more kinds.

Among these functional group-containing internal olefins and functional group-containing cyclic olefins, preferred are hydroxyl group-containing linear internal olefins such as 2-buten-1-ol [1-hydroxy-2-butene], 2-hexen-1-ol [1-hydroxy-2-hexene], 4-hexen-1-ol [1-hydroxy-4-hexene], 2-octen-1-ol [1-hydroxy-2-octene], 6-octen-1-ol [1-hydroxy-6-octene], 3-nonen-1-ol [1-hydroxy-3-nonene], and 6-nonen-1-ol [1-hydroxy-6-nonene]; cyano group-containing linear internal olefins such as 3-pentenenitrile [1-cyano-2-butene], 3-heptenenitrile [1-cyano-2-hexene], 5-heptenenitrile [1-cyano-4-hexene], 3-nonenenitrile [1-cyano-2-octene], 7-nonenenitrile [1-cyano-6-octene], 4-decenenitrile [1-cyano-3-nonene], and 7-decenenitrile [1-cyano-6-nonene]; alkoxy group-containing linear internal olefins such as 1-methoxy-2-butene, 1-methoxy-2-hexene, 1-methoxy-4-hexene, 1-methoxy-2-octene, 1-methoxy-6-octene, 1-methoxy-3-nonene, 1-methoxy-6-nonene, 1-benzyloxy-2-butene [1-phenylmethyloxy-2-butene], 1-benzyloxy-2-hexene [1-phenylmethyloxy-2-hexene], 1-benzyloxy-4-hexene [1-phenylmethyloxy-4-hexene], 1-benzyloxy-2-octene [1-phenylmethyloxy-2-octene], 1-benzyloxy-6-octene [1-phenylmethyloxy-6-octene], 1-benzyloxy-3-nonene [1-phenylmethyloxy-3-nonene], and 1-benzyloxy-6-nonene [1-phenylmethyloxy-6-nonene]; acetoxyl group-containing linear internal olefins such as 2-butenyl-1-acetate [1-acetoxy-2-butene], 2-hexenyl-1-acetate [1-acetoxy-2-hexene], 4-hexenyl-1-acetate [1-acetoxy-4-hexene], 2-octenyl-1-acetate [1-acetoxy-2-octene], 6-octenyl-1-acetate [1-acetoxy-6-octene], 3-nonenyl-1-acetate [1-acetoxy-3-nonene], and 6-nonenyl-1-acetate [1-acetoxy-6-nonene]; and oxo group-containing cyclic olefins such as 2-cyclohexen-1-one, from the viewpoint that the yield of the corresponding ketone having the functional group (hereinafter simply referred to as a "functional group-containing ketone") and the selectivity thereto are increased.

In the manufacturing method of the present invention, a concentration of the functional group-containing internal olefin or the functional group-containing cyclic olefin is preferably 0.01 to 5 mol/L, and more preferably 0.05 to 1 mol/L. If the concentration of the functional group-containing internal olefin or the functional group-containing cyclic olefin is lower than the lower limit, the functional group-containing ketone tends not to be obtained at a high yield. Meanwhile, if the concentration exceeds the upper limit, the functional group-containing ketone tends not to be manufactured at a high yield because the oxidation reaction of the functional group-containing internal olefin or the functional group-containing cyclic olefin does not proceed sufficiently.

### <Palladium catalyst>

The palladium catalyst used in the present invention is not particularly limited, as long as the palladium catalyst is a compound containing a palladium atom. Those which have been used in manufacturing of ketones can be each used as a palladium catalyst used in the present invention. Specific examples of such a palladium catalyst include palladium halides such as palladium chloride and palladium bromide, palladates such as sodium tetrachloropalladate, sodium tetrabromopalladate, potassium tetrachloropalladate, and potassium tetrabromopalladate, ammine complexes of palladium halides such as tetraamminepalladium dichloride and diamminepalladium tetrachloride, organic acid salts of palladium such as palladium acetate and palladium(II) trifluoroacetate, nitrile complexes of palladium halides such as diacetonitrile palladium dichloride and dibenzonitrile palladium dichloride, organic palladium compounds and complexes such as tris(dibenzylideneacetone) dipalladium-chloroform adduct and cyclooctadiene palladium dichloride, and the like. Moreover, anhydrides and hydrates of these compounds can be each used as the palladium catalyst. These palladium catalysts may be used singly or in combination of two or more kinds.

Among these palladium catalysts, preferred are palladium halides, and nitrile complexes of palladium halides, and more preferred are palladium halides, from the viewpoint that the yield and the selectivity can be improved in the oxidation reaction of the functional group-containing internal olefin or the functional group-containing cyclic olefin.

In the present invention, the palladium catalyst may be in a form of being dissolved in the amide-based solvent to be described later, or in a form of being uniformly or non-uniformly dispersed therein, or in a form of a combination thereof. For example, some of the components of the palladium catalyst (for example, the ligand) may be dissolved in the amide-based solvent, and the rest of the components may be uniformly or non-uniformly dispersed therein.

Moreover, in the present invention, a concentration of the palladium catalyst is preferably 0.001 to 1 mol/L, more preferably 0.002 to 0.5 mol/L, and particularly preferably 0.005 to 0.05 mol/L. If the concentration of the palladium catalyst is lower than the lower limit, the functional group-containing ketone tends not to be manufactured at a high yield, because the oxidation reaction of the functional group-containing internal olefin or the functional group-containing cyclic olefin does not proceed sufficiently. Meanwhile, if the concentration of the palladium catalyst exceeds the upper limit, the oxidation reaction of the functional group-containing internal olefin or the functional group-containing cyclic olefin tends not to proceed sufficiently because of the formation of an inactive species, Pd black.

### <Amide-based solvent>

In the present invention, the amide-based solvent represented by the formula (1) is used as a solvent. The use of such an amide-based solvent makes it possible to efficiently reoxidize the palladium catalyst with molecular oxygen.

In the formula (1), R¹ represents an alkyl group having 1 to 4 carbon atoms, and R² and R³ each independently represent an alkyl group having 1 to 4 carbon atoms or an aryl group. When R¹ and R² are alkyl groups, R¹ and R² may be bonded to each other to form a ring structure. Examples of such a ring structure include the pyrrolidone skeleton, the caprolactam skeleton, and the like.

Specific examples of the amide-based solvent used in the present invention include N,N-dimethylacetamide, N,N-diethylacetamide, N,N-dipropylacetamide, N-methyl-N-ethylacetamide, N-butyl-N-phenylacetamide, N,N-dimethylpropanamide, N,N-diethylpropanamide, N-methyl-N-ethylpropanamide, N-methyl-2-pyrrolidone, N-methyl-2-caprolactam, N-ethyl-2-caprolactam, and the like. These solvents may be used singly or in combination of two or more kinds. In addition, in the present invention, the amide-based solvent may be used in combination with other solvents.

Among the amide-based solvents, preferred are N,N-dimethylacetamide and N-methyl-2-pyrrolidone, and more preferred are N,N-dimethylacetamide, from the viewpoint that the yield and the selectivity are improved in the oxidation reaction of the functional group-containing internal olefin or the functional group-containing cyclic olefin.

In the present invention, the amount of the amide-based solvent used is set as appropriate so that the concentrations of the functional group-containing internal olefin or the functional group-containing cyclic olefin and the palladium catalyst can be within the above-described ranges.

### <Water>

In the present invention, the functional group-containing ketone is manufactured by allowing the functional group-containing internal olefin or the functional group-containing cyclic olefin to react with water. The amount of water added is not particularly limited, as long as the amount is necessary for the reaction. The amount of water may be set as appropriate according to the kinds of the functional group-containing internal olefin or the functional group-containing cyclic olefin, the palladium catalyst, and the amide-based solvent to be used, as well as the reaction mode, and conditions thereof. Specifically, the amount of water is preferably 0.5 to 70 parts by volume, and more preferably 1 to 50 parts by volume, relative to 100 parts by volume of the amide-based solvent. If the amount of water added is less than the lower limit, the yield of the functional group-containing ketone tends to decrease because a sufficient oxidation reaction rate cannot be achieved. Meanwhile, if the amount of water exceeds the upper limit, the activity of the catalyst tends to be lowered because the palladium component deposits or aggregates as palladium metal. Moreover, the yield of the functional group-containing ketone tends to be decreased, because the low solubility of the functional group-containing internal olefin or the functional group-containing cyclic olefin in water leads to a decreased contact efficiency of the functional group-containing internal olefin or the functional group-containing cyclic olefin with the palladium catalyst, so that a sufficient oxidation reaction rate cannot be achieved.

### <Oxygen>

In the present invention, the palladium catalyst having been used to oxidize the functional group-containing internal olefin or the functional group-containing cyclic olefin is reoxidized by using molecular oxygen. At this time, since substantially no co-catalyst such as a copper catalyst is used, the oxidation reaction of the functional group-containing internal olefin or the functional group-containing cyclic olefin is not inhibited by the copper catalyst, so that the functional group-containing ketone can be manufactured from the functional group-containing internal olefin or the functional group-containing cyclic olefin at a high yield and a high selectivity.

Examples of the source of the above-described molecular oxygen include oxygen gas, oxygen-enriched air, air, mixture gases of oxygen gas with a diluent gas (these are collectively referred to as "oxygen-containing gases"). Examples of the diluent gas include nitrogen gas, helium gas, argon gas, carbon dioxide, and the like. Nitrogen gas is generally used as the diluent gas.

In the present invention, gases other than these oxygen-containing gases and than the diluent gases can be each used in combination, unless the effect of the present invention is impaired. Moreover, such an oxygen-containing gas may be supplied as a mixture with water, the amide-based solvent, or the like, as needed.

In the present invention, the oxygen-containing gas is preferably supplied at an oxygen pressure of 0.1 to 1 MPa (more preferably 0.3 to 1 MPa). If the oxygen pressure is lower than the lower limit, the functional group-containing ketone tends not to be manufactured at a high yield because of the formation of an inactive species, Pd black. Meanwhile, if the oxygen pressure exceeds the upper limit, in some cases an oxidized by-product is formed in some functional group-containing internal olefins and some functional group-containing cyclic olefins.

### <Oxidation reaction>

In the method for manufacturing a ketone of the present invention, the functional group-containing ketone is formed by oxidizing the functional group-containing internal olefin or the functional group-containing cyclic olefin in the amide-based solvent in the presence of water, the palladium catalyst, and molecular oxygen, thereby bonding an oxo group (=O) to at least one of the carbon atoms constituting the C=C bond, without oxidizing the functional group in the functional group-containing olefin. Note that, in this description, such a functional group-containing ketone is referred to as a "corresponding ketone having a functional group" or the like.

In the present invention, the mode of the oxidation reaction is not particularly limited, as long as the palladium catalyst and the functional group-containing internal olefin or the functional group-containing cyclic olefin can be brought into contact with each other. For example, the oxidation reaction can be conducted in any form of gas-liquid reaction and/or liquid-liquid reaction according to the functional group-containing internal olefin or the functional group-containing cyclic olefin, and the palladium catalyst to be used. Moreover, a batch, semi-batch, semi-continuous, or continuous-flow reaction system, or a combination thereof can be employed. In addition, the method for supplying components such as the functional group-containing internal olefin or the functional group-containing cyclic olefin is not particularly limited, and the components may be supplied in a form of liquid or a form of gas.

Specific examples of the manufacturing method include a batch method in which the oxygen-containing gas and a catalyst solution prepared by mixing the palladium catalyst with the amide-based solvent or a mixture solution obtained by mixing the functional group-containing internal olefin or the functional group-containing cyclic olefin with the catalyst solution are placed in a batch reactor, and allowed to react with each other; a semi-batch method or a semi-continuous method in which the functional group-containing internal olefin or the functional group-containing cyclic olefin and the oxygen-containing gas are continuously fed into the catalyst solution, or the oxygen-containing gas is continuously fed into the mixture solution; a continuous-flow method in which the catalyst solution, the functional group-containing internal olefin or the functional group-containing cyclic olefin, and the oxygen-containing gas are caused to flow simultaneously through a reaction region; and the like.

In the present invention, when the functional group-containing internal olefin or the functional group-containing cyclic olefin and the oxygen-containing gas are continuously fed into the catalyst solution, the feed rate of the functional group-containing internal olefin or the functional group-containing cyclic olefin is preferably 10 to 5000 mol/h per mole of palladium. If the feed rate of the functional group-containing internal olefin or the functional group-containing cyclic olefin is lower than the lower limit, the amount of the functional group-containing ketone produced per unit time tends to decrease. Meanwhile, if the feed rate exceeds the upper limit, the functional group-containing ketone tends not to be obtained at a high yield because of the formation of an inactive species, Pd Black. Note that the feed rate of the oxygen-containing gas is adjusted as appropriate so that the oxygen pressure inside the reaction system is within the above-described range.

In the present invention, the reaction temperature for carrying out the oxidation reaction is preferably 0 to 200°C, and more preferably 20 to 100°C. If the reaction temperature is lower than the lower limit, the yield of the functional group-containing ketone tends to be decreased because of a slow reaction rate. Meanwhile, if the reaction temperature exceeds the upper limit, the selectivity to the functional group-containing ketone tends to be decreased because a side-reaction, such as isomerization, of the functional group-containing internal olefin occurs.

Moreover, in the present invention, a concentration of a copper catalyst, which is used in the conventional Wacker process, is preferably 0.03 mol/L or less, more preferably 0.01 mol/L or less, and particularly preferably 0.003 mol/L or less. If the concentration of the copper catalyst exceeds the upper limit, the yield of the functional group-containing ketone tends to be decreased. From such a viewpoint, in the present invention, it is most preferable to oxidize the functional group-containing internal olefin or the functional group-containing cyclic olefin in the absence of any copper catalyst. In the conventional Wacker process, the copper catalyst accelerates the reoxidation of the palladium catalyst. On the other hand, in a Wacker reaction of a functional group-containing olefin, such as the reaction of the present invention, the yield of the functional group-containing ketone tends to be decreased if a copper catalyst is coexistent. Accordingly, it is presumed that the copper catalyst inhibits an activity of the palladium catalyst in the reaction which would proceed efficiently with molecular oxygen unless the copper catalyst is coexistent.

The functional group-containing ketone thus obtained can be obtained as any one of a single compound and a mixture, which have a desired purity or composition, by separating and purifying the ketone in a usual manner. In the manufacturing method of the present invention, since few side reactions occur during the oxidation reaction, the unreacted raw material can be recovered and reused for manufacturing the ketone. The amide-based solvent and the palladium catalyst can also be separated and recovered, and then used repeatedly. At this time, the palladium catalyst can be appropriately regenerated, if necessary.

### [Examples]

Hereinafter, the present invention will be described more specifically on the basis of Examples and Comparative Examples. However, the present invention is not limited to the examples below.

### (Example 1)

Palladium chloride (manufactured by N. E. Chemcat Corporation, 17.6 mg, 0.1 mmol), N,N-dimethylacetamide (DMA, 5 ml), and water (0.5 ml) were placed in a pressure vessel, and heated to 80°C to dissolve palladium chloride. The obtained solution was transferred to a stainless steel autoclave reactor equipped with an inner tube made of Teflon (registered trademark). Then, the pressure inside the reactor was raised to 0.9 MPa by supplying oxygen gas thereto, and stirring was conducted at room temperature for 1 hour. Subsequently, the pressure inside the reactor was released, and 6-nonen-1-ol [1-hydroxy-6-nonene] (142 mg, 1.0 mmol) was added thereto. Then, the pressure inside the reactor was raised to 0.3 MPa by supplying oxygen gas thereto, and the oxidation reaction was allowed to proceed at 80°C for 10 hours.

The solution after completion of the reaction was analyzed by using a gas chromatograph equipped with a FID detector ("GC-2014" manufactured by Shimadzu Corporation, column: KOCL (3 m)), with naphthalene being employed as an internal standard substance. As a result, it was found that 1-hydroxy-6-nonanone [6-nonanon-1-ol] and 1-hydroxy-7-nonanone [7-nonanon-1-ol] were formed. Accordingly, 6-nonen-1-ol was presumably oxidized by bonding oxo group (=O) to a carbon atom in the C=C bond, without oxidizing the hydroxyl group, as shown in the following reaction formula (I): 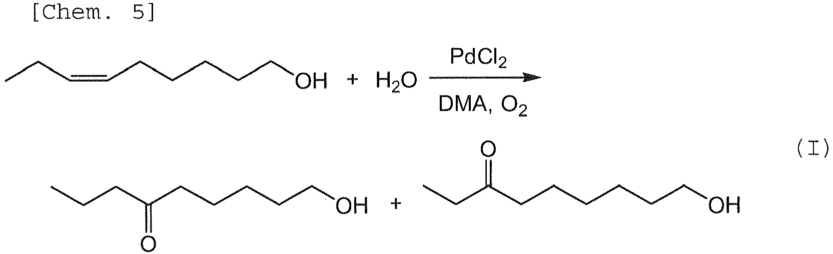 Table 1 shows the conversion of 6-nonen-1-ol and the selectivities to 1-hydroxy-6-nonanone and 1-hydroxy-7-nonanone among all the products.

Next, the solution after completion of the reaction was subjected to two extraction treatment by using a 1:1 mixture solution (30 ml) of diethyl ether and aqueous sodium chloride, and the diethyl ether layer was dried by MgSO₄, filtered, and then vacuum concentrated. The obtained crude reaction product was purified by column chromatography (packing: silica gel, eluent: a mixture solution of ethyl acetate/hexane (1:4)). Table 1 shows the isolated yield in this case.

### (Example 2)

An oxidation reaction was conducted in the same manner as in Example 1, except that 6-nonen-1-ol was replaced with 3-pentenenitrile [1-cyano-2-butene] (81 mg, 1.0 mmol).

The solution after completion of the reaction was analyzed by using a gas chromatograph equipped with a FID detector ("GC-2014" manufactured by Shimadzu Corporation, column: KOCL (3 m)), with naphthalene being employed as an internal standard substance. As a result, it was found that 1-cyano-3-butanone [4-oxo-pentanenitrile] was formed. Accordingly, 3-pentenenitrile was presumably oxidized by bonding oxo group (=O) to a carbon atom in the C=C bond, without oxidizing the cyano group, as shown in the following reaction formula (II): 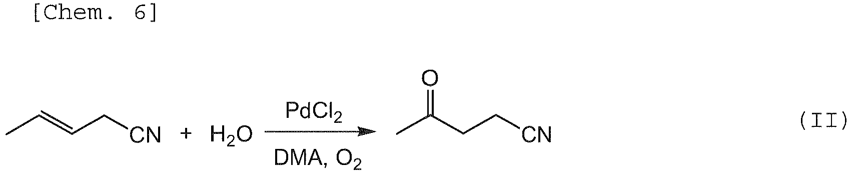 Table 1 shows the conversion of 3-pentenenitrile and the selectivity to 1-cyano-3-butanone among all the products.

Next, a crude reaction product was collected from the solution after completion of the reaction, and purified, in the same manner as in Example 1. Table 1 shows the isolated yield in this case. In addition, the purified reaction product was identified as 1-cyano-3-butanone by ¹H-NMR analysis and ¹³C-NMR analysis. The ¹H-NMR and ¹³C-NMR chemical shifts of 1-cyano-3-butanone are shown below. ¹H-NMR (270 MHz, CDCl₃) δ 2.84 (t, J=6.9 Hz, 2H), 2.57 (t, J=6.9 Hz, 2H), 2.22 ppm (s, 3H). ¹³C-NMR (68 MHz, CDCl₃) δ 203.4, 118.8, 38.51, 29.42, 11.34 ppm.

### (Example 3)

An oxidation reaction was conducted in the same manner as in Example 1, except that 6-nonen-1-ol was replaced with 1-benzyloxy-2-hexene [1-phenylmethyloxy-2-hexene] (190 mg, 1.0 mmol).

The solution after completion of the reaction was analyzed by using a gas chromatograph equipped with a FID detector ("GC-2014" manufactured by Shimadzu Corporation, column: KOCL (3 m)), with naphthalene being employed as an internal standard substance. As a result, it was found that 1-benzyloxy-3-hexanone [1-phenylmethyloxy-3-hexanonel was formed. Accordingly, 1-benzyloxy-2-hexene was presumably oxidized by bonding oxo group (=O) to a carbon atom in the C=C bond, without oxidizing the benzyloxy group, as shown in the following reaction formula (III): 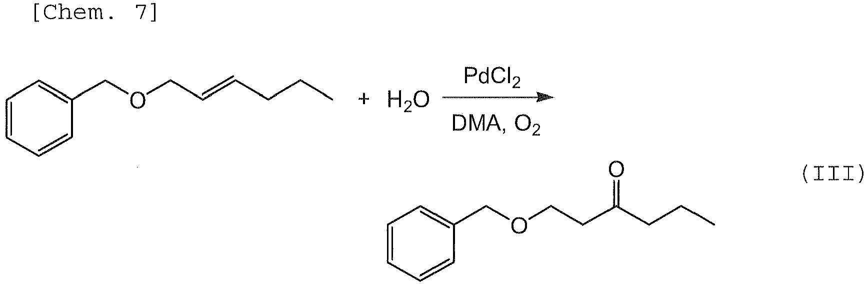 Table 1 shows the conversion of 1-benzyloxy-2-hexene and the selectivity to 1-benzyloxy-3-hexanone among all the products.

Next, a crude reaction product was collected from the solution after completion of the reaction, and purified, in the same manner as in Example 1. The purified reaction product was identified as 1-benzyloxy-3-hexanone by ¹H-NMR analysis, ¹³C-NMR analysis and HRMS analysis. The ¹H-NMR and ¹³C-NMR chemical shifts and the result of the HRMS analysis of 1-benzyloxy-3-hexanone are shown below. ¹H-NMR (270 MHz, CDCl₃) δ 7.22-7.37 (m, 5H), 4.50 (s, 2H), 3.74 (t, J=6.3 Hz, 2H), 2.68 (t, J=6.3 Hz, 2H), 2.42 (t, J=7.3 Hz, 2H), 1.61 (sextet, J=7.3 Hz, 2H), 0.91 ppm (t, J=7.3 Hz, 3H).

¹³C-NMR (68 MHz, CDCl₃) δ 209.0, 138.1, 128.3 (2 signals), 127.6 (2 signals), 127.5, 73.2, 65.4, 45.4, 42.9, 17.1, 13.8 ppm.

HRMS (EI) (m/z) , calcd for C₁₃H₁₈O₂, 206.2808; found, 206.2847.

### (Example 4)

An oxidation reaction was conducted in the same manner as in Example 1, except that 6-nonen-1-ol was replaced with 1-methoxy-2-octene (142 mg, 1.0 mmol).

The solution after completion of the reaction was analyzed by using a gas chromatograph equipped with a FID detector ("GC-2014" manufactured by Shimadzu Corporation, column: KOCL (3 m)), with naphthalene being employed as an internal standard substance. As a result, it was found that 1-methoxy-3-octanone was formed. Accordingly, 1-methoxy-2-octene was presumably oxidized by bonding oxo group (=O) to a carbon atom in the C=C bond, without oxidizing the methoxy group, as shown in the following reaction formula (IV): 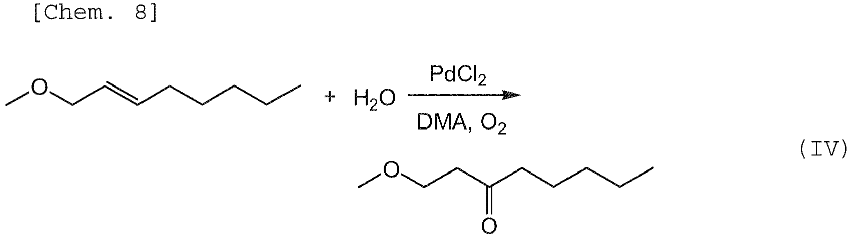 Table 1 shows the conversion of 1-methoxy-2-octene and the selectivity to 1-methoxy-3-octanone among all the products.

Next, a crude reaction product was collected from the solution after completion of the reaction, and purified, in the same manner as in Example 1. The purified reaction product was identified as 1-methoxy-3-octanone by ¹H-NMR analysis and ¹³C-NMR analysis. The ¹H-NMR and ¹³C-NMR chemical shifts of 1-methoxy-3-octanone are shown below. ¹H-NMR (270 MHz, CDCl₃) δ 3.64 (t, J=6.3 Hz, 2H), 3.33 (s, 3H), 2.65 (t, J=6.3 Hz, 2H), 2.43 (t, J=7.6 Hz), 1.58 (quintet, J=7.3 Hz, 2H), 1.24-1.31 (m, 4H), 0.89 ppm (t, J=6.9 Hz, 3H).

¹³C-NMR (68 MHz, CDCl₃) δ 209.1, 67.7, 58.8, 43.5, 42.8, 31.4, 23.4, 22.5, 14.0 ppm.

### (Example 5)

An oxidation reaction was conducted in the same manner as in Example 1, except that 6-nonen-1-ol was replaced with 2-butenyl-1-acetate [1-acetoxy-2-butene] (57 mg, 0.5 mmol) and that the reaction time was changed to 6 hours.

The solution after completion of the reaction was analyzed by using a gas chromatograph equipped with a FID detector ("GC-2014" manufactured by Shimadzu Corporation, column: KOCL (3 m)), with naphthalene being employed as an internal standard substance. As a result, it was found that 1-acetoxy-3-butanone [3-butanone-1-acetate] and 1-acetoxy-2-butanone [2-butanone-1-acetate] were formed. Accordingly, 2-butenyl-1-acetate was presumably oxidized by bonding oxo group (=O) to a carbon atom in the C=C bond, without oxidizing the acetoxyl group, as shown in the following reaction formula (V): 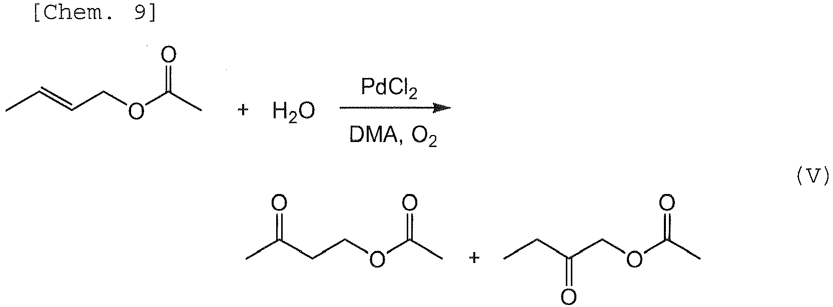 Table 1 shows the conversion of 2-butenyl-1-acetate and the selectivities to 1-acetoxy-3-butanone and 1-acetoxy-2-butanone among all the products.

### (Example 6)

An oxidation reaction was conducted in the same manner as in Example 5, except that the amount of palladium chloride supplied was changed to 31.9 mg (0.18 mmol), the amount of N,N-dimethylacetamide (DMA) supplied was changed to 15 ml, the amount of water supplied was changed to 1.5 ml, and the amount of 2-butenyl-1-acetate supplied was changed to 128.8 mg (1.13 mmol), and that the pressure inside the reactor was raised to 0.6 MPa.

The solution after completion of the reaction was analyzed by using a gas chromatograph equipped with a FID detector ("GC-2014" manufactured by Shimadzu Corporation, column: KOCL (3 m)), with naphthalene being employed as an internal standard substance. As a result, it was found that 1-acetoxy-3-butanone and 1-acetoxy-2-butanone were formed. Table 1 shows the conversion of 2-butenyl-1-acetate and the selectivities to 1-acetoxy-3-butanone and 1-acetoxy-2-butanone among all the products.

### (Example 7)

An oxidation reaction was conducted in the same manner as in Example 6, except that 2-butenyl-1-acetate was replaced with 2-buten-1-ol [1-hydroxy-2-butene] (113.5 mg, 1.58 mmol).

The solution after completion of the reaction was analyzed by using a gas chromatograph equipped with a FID detector ("GC-2014" manufactured by Shimadzu Corporation, column: KOCL (3 m)), with naphthalene being employed as an internal standard substance. As a result, it was found that 1-hydroxy-3-butanone [3-butanon-1-ol] and 1-hydroxy-2-butanone [2-butanon-1-ol] were formed. Accordingly, 2-buten-1-ol was presumably oxidized by bonding oxo group (=O) to a carbon atom in the C=C bond, without oxidizing the hydroxyl group, as shown in the following reaction formula (VI): 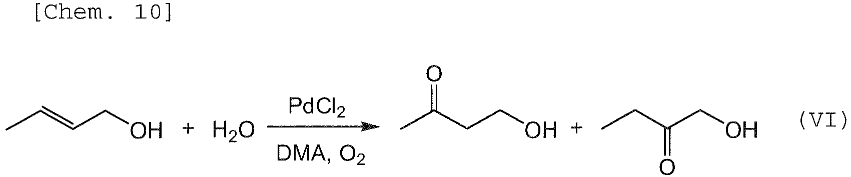 Table 1 shows the conversion of 2-buten-1-ol and the selectivities to 1-hydroxy-3-butanone and 1-hydroxy-2-butanone among all the products.

### (Example 8)

An oxidation reaction was conducted in the same manner as in Example 5, except that 2-butenyl-1-acetate was replaced with 2-cyclohexen-1-one (486.1 mg, 5.0 mmol), and that the amount of palladium chloride supplied was changed to 180.3 mg (1.0 mmol), the amount of N,N-dimethylacetamide (DMA) supplied was changed to 50 ml, and the amount of water supplied was changed to 5.0 ml.

The solution after completion of the reaction was analyzed by using a gas chromatograph equipped with a FID detector ("GC-2014" manufactured by Shimadzu Corporation, column: KOCL (3 m)), with naphthalene being employed as an internal standard substance. As a result, it was found that 1,3-cyclohexadione, phenol, and resorcinol were formed. Accordingly, 2-cyclohexen-1-one was presumably oxidized by bonding oxo group (=O) to a carbon atom in the C=C bond, without oxidizing the original hydroxyl group, as shown in the following reaction formula (VII): 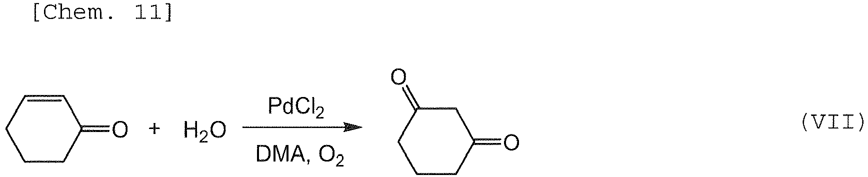 Table 1 show the conversion of 2-cyclohexen-1-one and the selectivity to 1,3-cyclohexadione among all the products.

### (Comparative Example 1)

An oxidation reaction was conducted in the same manner as in Example 6, except that N,N-dimethylacetamide (DMA) was replaced with acetonitrile (AcCN, 15 ml).

The solution after completion of the reaction was analyzed by using a gas chromatograph equipped with a FID detector ("GC-2014" manufactured by Shimadzu Corporation, column: KOCL (3 m)), with naphthalene being employed as an internal standard substance. As a result, it was found that a trace amount of 1-acetoxy-3-butanone was formed. Table 1 shows the conversion of 2-butenyl-1-acetate and the selectivity to 1-acetoxy-3-butanone among all the products.

**[Table 1]**

| | Olefin | Catalyst | Solvent | Ketone | Conversion (%) | Selectivity (%) | Isolated yield (%) |
|---|---|---|---|---|---|---|---|
| Ex. 1 | 6-nonen-1-ol | PdCl₂ | DMA | 1-hydroxy-6-nonanone | 92 | 49 | 81 |
| | | | | 1-hydroxy-7-nonanone | | 51 | |
| Ex. 2 | 3-pentenenitrile | PdCl₂ | DMA | 1-cyano-3-butanone | 93 | 99 | 86 |
| Ex. 3 | 1-benzyloxy-2-hexene | PdCl₂ | DMA | 1-benzyloxy-3-hexanone | 95 | 96 | - |
| Ex. 4 | 1-methoxy-2-octene | PdCl₂ | DMA | 1-methoxy-3-octanone | 85 | 94 | - |
| Ex. 5 | 2-butenyl-1-acetate | PdCl₂ | DMA | 1-acetoxy-3-butanone | 90 | 99 | - |
| | | | | 1-acetoxy-2-butanone | | 1 | |
| Ex. 6 | 2-butenyl-1-acetate | PdCl₂ | DMA | 1-acetoxy-3-butanone | 100 | 98.1 | - |
| | | | | 1-acetoxy-2-butanone | | 1.9 | |
| Ex. 7 | 2-buten-1-ol | PdCl₂ | DMA | 1-hydroxy-3-butano ne | 100 | 86.1 | - |
| | | | | 1-hydroxy-2-buta no ne | | 13.9 | |
| Ex. 8 | 2-cyclohexen-1-one | PdCl₂ | DMA | 1,3-cyclohexadione | 86.7 | 59.9 | - |
| Comp. Ex. 1 | 2-butenyl-1-acetate | PdCl₂ | AcCN | 1-acetoxy-3-butanone | 1.1 | 100 | - |

As is apparent from the results shown in Table 1, it was found that, by oxidizing an internal olefin or a cyclic olefin having a functional group such as hydroxyl group in the presence of a palladium catalyst, water, and molecular oxygen by use of a specific amide-based solvent as a solvent, a functional group-containing ketone corresponding to the internal olefin or the cyclic olefin was successfully manufactured at a high yield and a high selectivity, without oxidizing the functional group.

In addition, in the cases (Examples 2 to 7) where an internal olefin having a functional group at an allylic position was oxidized, the oxo group was bonded to the carbon atom more distant from the functional group, of the two carbon atoms constituting the C=C bond, at a high selectivity of 85% or higher. It was found that the method for manufacturing a ketone of the present invention was capable of oxidizing an internal olefin having a functional group at an allylic position with a high positional selectivity, and thereby manufacturing a corresponding ketone having the functional group. It was found that, particularly in the case where the functional group is at least one selected from the group consisting of cyano group, alkoxy groups, and acetoxyl groups, the oxo group was bonded to the carbon atom more distant from the functional group, of the two carbon atoms constituting the C=C bond, at an extremely high selectivity of 95% or higher.

In contrast, in the case (Comparative Example 1) where acetonitrile was used as the solvent, it was found that the conversion was as low as 1.1%, indicating that the oxidation reaction hardly proceeded.

### [Industrial Applicability]

As has been described above, the present invention makes it possible to manufacture a corresponding ketone having a functional group such as hydroxyl group at a high yield and a high selectivity from an internal olefin or a cyclic olefin having the functional group.

Accordingly, the method for manufacturing a ketone of the present invention is economically advantageous because of the high yield of the corresponding ketone having the functional group and the high selectivity thereto, and the ketones having the functional group obtained by the above-described method are useful as industrial raw materials such as solvents and chemical raw materials. Moreover, 1-acetoxy-3-butanone, 1-hydroxy-3-butanone, and the like are precursors of 1,3-butanediol used as a raw material of polyesters and as a moisturizing agent. In addition, 1,3-cyclohexane dione and the like have high potential in industry, because 1,3-cyclohexane dione and the like are an intermediate of herbicides (sulcotrione and mesotrione) or agricultural chemicals, and is used in various organic syntheses.

## Claims

1. A method for manufacturing a ketone, comprising:
oxidizing an internal olefin or a cyclic olefin having a functional group containing a hetero atom and one carbon-carbon double bond or more at a position other than terminals of a molecule thereof in an amide-based solvent in the presence of water, a palladium catalyst, and molecular oxygen, without oxidizing the functional group, thereby bonding an oxo group to at least one of the carbon atoms constituting the carbon-carbon double bond, the amide-based solvent being represented by the following formula (1): 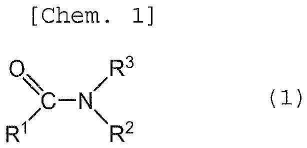 (in the formula (1), R¹ represents an alkyl group having 1 to 4 carbon atoms; R² and R³ each independently represent an alkyl group having 1 to 4 carbon atoms or an aryl group;
and when R¹ and R² are alkyl groups, R¹ and R² may be bonded to each other to form a ring structure).

2. The method for manufacturing a ketone according to claim 1, wherein the palladium catalyst is a palladium halide.

3. The method for manufacturing a ketone according to claim 1 or 2, wherein the internal olefin or the cyclic olefin is a compound represented by the following formula (2) : 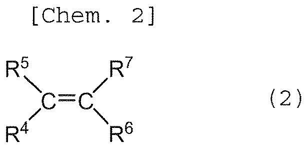 (in the formula (2), at least one of R⁴ to R⁷ is one selected from the group consisting of alkyl groups having the functional group, alkenyl groups having the functional group, and aryl groups having the functional group; the rest of R⁴ to R⁷ are each independently one selected from the group consisting of a hydrogen atom, alkyl groups, alkenyl groups, and aryl groups; at least one of R⁴ and R⁵ is a group other than a hydrogen atom; at least one of R⁶ and R⁷ is a group other than a hydrogen atom; when R⁴ and R⁶ are an alkyl group or an alkenyl group, R⁴ and R⁶ may be bonded to each other to form a ring structure; and when R⁵ and R⁷ are an alkyl group or an alkenyl group, R⁵ and R⁷ may be bonded to each other to form a ring structure).

4. The method for manufacturing a ketone according to any one of claims 1 to 3, wherein the internal olefin or the cyclic olefin has the functional group which is bonded to a first to third carbon atom away from the carbon-carbon double bond.

5. The method for manufacturing a ketone according to any one of claims 1 to 4, wherein the functional group in the internal olefin or the cyclic olefin is at least one selected from the group consisting of hydroxyl group, cyano group, alkoxy groups, acetoxyl groups, and oxo group.

6. The method for manufacturing a ketone according to any one of claims 1 to 5, wherein the internal olefin or the cyclic olefin does not have any carbon-carbon double bond at the terminals of the molecule thereof.

7. The method for manufacturing a ketone according to any one of claims 1 to 6, wherein the amide-based solvent is N,N-dimethylacetamide.

8. The method for manufacturing a ketone according to any one of claims 1 to 7, wherein the internal olefin or the cyclic olefin is oxidized in the absence of any copper catalyst.

9. The method for manufacturing a ketone according to any one of claims 1 to 8, wherein a concentration of the palladium catalyst is 0.001 to 1 mol/L.
